# EUROPEAN PATENT APPLICATION

(11) **EP 2 591 809 A1**
(43) Date of publication of application: **15.05.2013**
(21) Application number: 12192143.1
(22) Date of filing: 09.11.2012
(51) Int. Cl.: A61L 9/12, B65D 83/00

(54) **Device for releasing substances to the atmosphere**

(30) Priority: 10.11.2011 ES 201101100 U
(71) Applicant: Zyxtudio diseño e innovación SL, 46003 Valencia (ES)
(72) Inventor: BLASCO FEO, VICENTE, 46003 Valencia (ES)
(74) Representative: Soler Lerma, Santiago

(57) **Abstract**

Device for releasing substances to the atmosphere of the type comprising at least one deposit in which the substance to release is contained, this substance being in contact with a breathable membrane that allows the gradual release of the substance, the device comprising a laminar body with an internal through orifice, such as in an O shape, open in one of its bases and having a folding area on the opposite base.

## Description

The present invention, as its title indicates, relates to a device for releasing substances to the atmosphere of the type comprising at least one deposit in which the substance to release is contained, this substance being in contact with a breathable membrane that allows the gradual release of the substance, in this case the device comprising a laminar body with an internal through orifice, such as in an O shape, open in one of its bases and having a folding area on the opposite base.

This invention belongs to the field of air fresheners and insecticides.

### BACKGROUND OF THE INVENTION

This party has no reference of similar antecedents, although there are multiple devices that use breathable membranes in contact with the liquid contained in a deposit as a means for volatising said liquids. Examples of this are patents WO2010/121039 or WO2010/120960, although both require a support for the deposit and means for breaking an additional wall.

On another hand, references have been found to devices that incorporate elements meant to be hung inside closets, or even some inserted in coat hangers, examples of which are Spanish Utility Mode U283273 relating to a coat hanger with recesses allowing the insertion of a moth repellent element, Utility Mode ES1008233U relating to a coat hanger with a hollow bottom bar that can house an air-freshening or moth repellent element, or Utility Model ES1063788U relating to a base body in which is defined a plurality of small recesses or dimples in which to house the corresponding insecticide pills, a hook-shaped extension being joined to said body in order to hang it in a rack or closet.

The proposed invention is simpler than all known embodiments and can also be hung in a closet or placed on a flat base, ensuring at all times that the substances are released.

### DESCRIPTION OF THE INVENTION

The proposed invention comprises a blister-type flexible laminar body with an internal through orifice, such as with an O shape, in which a relief defines at least one cavity or deposit filled with the substance to volatilise, a breathable membrane covering said laminar body on the part in which the relief is not present and a removable film that covers said breathable membrane to prevent releasing the substance before the device is in use.

The laminar body, such as with an O shape, has an opening that communicates it internal and external perimeter and a folding line.

The opening communicating the internal and external perimeter can be made when manufacturing it or the device can present a pre-cut line that the user rips to communicate the internal and external perimeter.

The device can be used in closets, where it can be hung independently without requiring a hook, or on flat surfaces.

To hook it inside a closet, such as to a bar, it is enough to slightly separate the ends that limit the opening communicating the internal and external perimeters.

When the device is embracing the bar its flexibility will make the bent ends return to their initial position, so that the device will be hanging from the bar.

To use the device on flat surfaces, it is advisable to bend it along the folding line, which is preferably in an axial direction.

When the device is bent the aeration of the membrane is ensured, and therefore also the release of the substance contained in it or in the deposits.

### BRIEF DESCRIPTION OF THE DRAWINGS

To aid a better understanding of the description, the following figures are provided that show an example of embodiment of the invention.

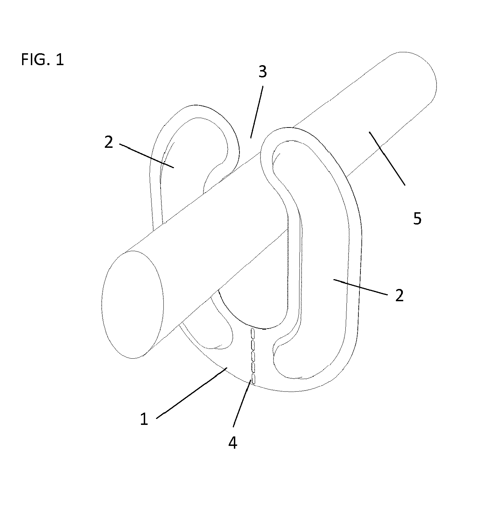

FIGURE 1 shows an embodiment of the device in an O shape that is open on the top, with the internal and external perimeters communicated, revealing the laminar body that forms the body of the device (1), the two deposits (2) configured in the laminar body, the top opening (3), the folding line (4), and the bar (5), which can be for example the bar of a closet, which although it does not form part of the invention, it allows explaining the form of connection.

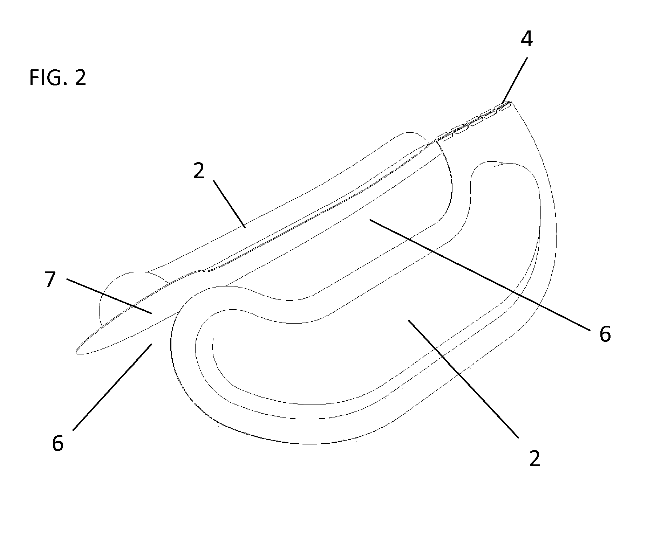

FIGURE 2 shows the invention in the position adopted for use on surfaces in which the device is bent along the folding line (4), defining under it a space (6) large enough to allow aeration and transpiration of the membrane (7) that covers the device on its bottom part and is in contact with the substance contained in the deposits (2).

### DESCRIPTION OF AN EMBODIMENT OF THE INVENTION

An example of an embodiment of the invention is described below that is not unique or limiting.

The device for releasing substances to the atmosphere comprises:
A laminar body of flexible material (1) with an O shape having an opening (3) that communicates the internal and external perimeters.

This laminar body is provided with two cavities (2), formed in the body itself, that run along its sides surrounding the internal orifice, which constitute the deposits for the material to be released such that there can be a different material in each one.

A breathable membrane (7) that covers at least the open part of said deposits.

A non-breathable film, preferably of aluminium, that covers at least the part of the opening of the cavities covered by the membrane.

An axial folding line (4) on the base opposite to that with the opening.

The blister-type laminar body (1) has deposits preferably made of transparent plastic, so that users can easily see the amount of substance remaining.

In a preferred embodiment of the invention, the face of the laminar body on which the deposits open is completely covered by the microporous membrane.

On top of the microporous membrane is adhered an easily removable aluminium film, so that during the transport and storage of the device this film will seal the deposits, with the membrane in between, and to use the device the aluminium film is removed so that the membrane is in contact with the atmosphere, thereby allowing the dispersion of the substances contained in the deposits.

Due to the flexibility of the material, the sides of the O covered by the deposits can move, one in each sense, with respect to the closed base of the device, so that the opening of the top base is enlarged so that it can embrace a bar (5) of the type found in closets to hang coat hangers, after which the sides return to their initial position and the device will be hanging from the bar, as shown in figure 1.

Another way to use the device is on a flat surface, for which it is advisable to bend it along the folding line so that it adopts the shape shown in figure 2, which favours the transpiration of the membrane (7) by generating an aeration space (6).

In this way, with the device in a horizontal position and the membrane in the bottom face, the membrane is prevented from touching and staining any clothes that it may be in contact with, while favouring the transpiration of the membrane.

If the device is turned over so that the membrane is in the top position, the square profile of the device with the membrane on its internal faces will also prevent the membrane from staining any clothing items that may be in contact with the device.

## Claims

1. DEVICE FOR RELEASING SUBSTANCES TO THE ATMOSPHERE of the type in which the substance to evaporate is contained in a deposit, with said substance in contact with a breathable membrane, **characterised in that** it comprises:
A flexible laminar body 81) surrounding an internal orifice;
A communication duct (3) between the internal and external perimeters of said laminar body;
At least one deposit (2) formed in the laminar body containing the substance to evaporate;
A breathable membrane (7) that covers the open part of said deposit and is in contact with said substance to evaporate;
A film covering said membrane.

2. DEVICE FOR RELEASING SUBSTANCES TO THE ATMOSPHERE according to claim 1, **characterised in that** the laminar body is symmetrical.

3. DEVICE FOR RELEASING SUBSTANCES TO THE ATMOSPHERE according to claim 1, **characterised in that** the communication duct between the internal and external perimeter of the device is an opening.

4. DEVICE FOR RELEASING SUBSTANCES TO THE ATMOSPHERE according to claim 1, **characterised in that** the communication duct between the internal and external perimeter of the device is pre-cut line.

5. DEVICE FOR RELEASING SUBSTANCES TO THE ATMOSPHERE according to claim 1, **characterised in that** it has a folding line (4).

6. DEVICE FOR RELEASING SUBSTANCES TO THE ATMOSPHERE according to claim 2, **characterised in that** it has a folding line (4) aligned axially with the device.

7. DEVICE FOR RELEASING SUBSTANCES TO THE ATMOSPHERE according to claim 1, **characterised in that** it has two deposits (2).

8. DEVICE FOR RELEASING SUBSTANCES TO THE ATMOSPHERE according to claim 7, **characterised in that** each deposit contains a different substance.

9. DEVICE FOR RELEASING SUBSTANCES TO THE ATMOSPHERE according to claim 1, **characterised in that** the deposit or deposits surround the internal orifice.
